# EUROPEAN PATENT APPLICATION

(11) **EP 1 411 054 A1**
(43) Date of publication of application: **21.04.2004**
(21) Application number: 03079072.9
(22) Date of filing: 19.04.2000
(51) Int. Cl.: C07D 417/12, A61K 31/427, A61P 3/10

(54) **Thiazolidinedione derivative and its use as antidiabetic**

(30) Priority: 20.04.1999 GB 9909075
(62) Divisional of application: 00920895.0
(71) Applicant: SMITHKLINE BEECHAM PLC, Brentford, Middlesex TW8 9GS (GB)
(72) Inventor: Blackler, Paul David James GlaxoSmithKline, Tonbridge Kent TN11 9AN (GB); Craig, Andrew Simon, GlaxoSmithKline, Tonbridge Kent TN11 9AN (GB); Giles, Robert Gordon, GlaxoSmithKline, Tonbridge Kent TN11 9AN (GB); Sasse, Michael John, GlaxoSmithKline, Tonbridge Kent TN11 9AN (GB)
(74) Representative: Rutter, Keith

(57) **Abstract**

5-[4-[2-(N-methyl-N-(2-pyridyl)amino)ethoxy]benzyl]thiazolidine-2,4-dione, hydrochloride monohydrate characterised in that it:
(i) provides an infra red spectrum containing peaks at 3358, 2764, 1245, 833 and 760cm⁻¹; and/or
(ii) provides an X-ray powder diffraction (XRPD) pattern containing peaks at 15.0, 17.7, 23.0, 30.0 and 31.4 °2θ; a process for preparing such a compound, a pharmaceutical composition containing such a compound and the use of such a compund in medicine.

## Description

This invention relates to a novel pharmaceutical, to a process for the preparation of the pharmaceutical and to the use of the pharmaceutical in medicine.

European Patent Application, Publication Number 0,306,228 relates to certain thiazolidinedione derivatives disclosed as having hypoglycaemic and hypolipidaemic activity. The compound of example 30 of EP 0,306,228 is 5-[4-[2-(N-methyl-N-(2-pyridyl)amino)ethoxy]benzyl]thiazolidine-2,4-dione (hereinafter also referred to as "Compound I").

International Patent Application, Publication Number WO94/05659 discloses certain salts of the compounds of EP 0,306,228 and in particular the maleic acid salt.

International Patent Applications, Publication Numbers WO99/31093, WO99/31094 and WO99/31095 each disclose distinct hydrates of Compound (I).

It has now been discovered that Compound I exists in the form of a novel hydrochloride salt which is monohydrated. This novel hydrochloride salt monohydrate (hereinafter also referred to as "Hydrochloride Hydrate") is particularly suitable for bulk preparation and handling. The novel form can be prepared by an efficient, economic and reproducible process particularly suited to large-scale preparation.

The novel Hydrochloride Hydrate also has useful pharmaceutical properties and in particular it is indicated to be useful for the treatment and/or prophylaxis of diabetes mellitus, conditions associated with diabetes mellitus and certain complications thereof.

Accordingly, the present invention provides 5-[4-[2-(N-methyl-N-(2-pyridyl)amino)ethoxy]benzyl]thiazolidine-2,4-dione, hydrochloride monohydrate characterised in that it:
(i) provides an infra red spectrum containing peaks at 3358, 2764, 1245, 833 and 760cm⁻¹; and/or
(ii) provides an X-ray powder diffraction (XRPD) pattern containing peaks at 15.0, 17.7, 23.0, 30.0 and 31.4 °2θ.

In one favoured aspect, Hydrochloride Hydrate provides an infra red spectrum substantially in accordance with Figure I. In one favoured aspect, Hydrochloride Hydrate provides an X-Ray powder diffraction pattern (XRPD) substantially in accordance with Figure II.

The Hydrochloride Hydrate may exist in certain dehydrated forms which reversibly convert to the Hydrochloride Hydrate when contacted with water, either in liquid or vapour form. The present invention encompasses all such reversibly rehydratable forms of the Hydrochloride Hydrate. Preferably, there is provided the hydrated form as characterised above.

The present invention encompasses Hydrochloride Hydrate isolated in pure form or when admixed with other materials.

Thus in one aspect there is provided Hydrochloride Hydrate in isolated form.

In a further aspect there is provided Hydrochloride Hydrate in pure form.

In yet a further aspect there is provided Hydrochloride Hydrate in crystalline form.

The invention also provides a process for preparing Hydrochloride Hydrate, characterised in that 5-[4-[2-(N-methyl-N-(2-pyridyl)amino)ethoxy]benzyl]thiazolidine-2,4-dione (Compound I) is treated with hydrochloric acid in acetic acid as solvent and thereafter the required compound is recovered.

Suitably the reaction is carried out at ambient temperature but any convenient temperature may be employed which provides the required product.

Recovery of the required compound generally comprises crystallisation using an appropriate solvent such as diethyl ether.

In a further process for preparing Hydrochloride Hydrate, a suspension of Compound (I) in a suitable aqueous organic solvent, preferably propan-2-ol, is treated with hydrochloric acid, preferably concentrated hydrochloric acid, at any temperature providing the required product such as a temperature in the range of from 20°C to 30 °C, preferably at ambient temperature. Preferably, the reaction mixture is agitated or stirred. Preferably, the total amount of water in the last above mentioned reaction is in the range of from about 2%w/v to about 20%w/v, for example 10%w/v.

The above-mentioned process may be seeded with Hydrochloride Hydrate, but this is not essential.

In a further process for preparing Hydrochloride Hydrate a solution of Compound (I) in a suitable organic solvent is reacted with hydrochloric acid to provide substantially dissolved reactants, crystallisation is then induced to provide the Hydrochloride Hydrate.

Preferably the total amount of water in the last above mentioned reaction is in the range of from about 2%w/v to about 20 %w/v, for example 10 %w/v

A suitable organic solvent is propan-2-ol or the like. A suitable organic solvent is acetonitrile or the like.

In one aspect of the above mentioned process, for example when the organic solvent is propan-2-ol or the like, substantially dissolved reactants are preferably obtained by carrying out the reaction at an elevated temperature, such as a temperature in the range of from 60°C to 80°C, suitably 65°C to 75°C, for example 70°C.

In one aspect of the above mentioned process for example when the organic solvent is acetonitrile or the like, substantially dissolved reactants are obtained by carrying out the reaction at ambient or elevated temperature, preferably ambient temperature.

In one aspect of the above mentioned process, for example when the organic solvent is propan-2-ol or the like, crystallisation is suitably induced by cooling the reaction, usually to ambient temperature, preferably in the presence of a seed of the Hydrochloride Hydrate.

In a further aspect of the above mentioned process, for example when the organic solvent is acetonitrile or the like, crystallisation is suitably induced by addition of an appropriate co-solvent such as diethyl ether, preferably at ambient temperature and preferably in the presence of a seed of the Hydrochloride Hydrate.

It will be appreciated that in the above mentioned processes hydrochloric acid may be replaced by any suitable source of hydrochloride ions, providing the amount of water in the reaction is suitable for formation of the of Hydrochloride Hydrate. The suitable amount of water is generally at least one molar equivalent and generally an excess over this, for example an amount equivalent to that used in the above mentioned processes.

Unless specified otherwise, crystallisation of the Hydrochloride Hydrate is generally carried out at low to ambient temperature, such as in the range of from 0 to 30°C for example 25°C; alternatively crystallisation may be initiated at an elevated temperature, such as in the range of from 30°C and 60°C for example 50°C, and then completed by allowing the temperature of the solvent to cool to ambient or low temperature, such as in the range of from 0 to 30°C for example 25°C. The crystallisation can be initiated by seeding with crystals of Hydrochloride Hydrate but this is not essential unless otherwise specified.

Compound (I) is prepared according to known procedures, such as those disclosed in EP 0,306,228 and WO94/05659. The disclosures of EP 0,306,228 and WO94/05659 are incorporated herein by reference.

When used herein the term 'prophylaxis of conditions associated with diabetes mellitus' includes the treatment of conditions such as insulin resistance, impaired glucose tolerance, hyperinsulinaemia and gestational diabetes.

Diabetes mellitus preferably means Type II diabetes mellitus.

Conditions associated with diabetes include hyperglycaemia and insulin resistance and obesity. Further conditions associated with diabetes include hypertension, cardiovascular disease, especially atherosclerosis, certain eating disorders, in particular the regulation of appetite and food intake in subjects suffering from disorders associated with under-eating , such as anorexia nervosa, and disorders associated with over-eating, such as obesity and anorexia bulimia. Additional conditions associated with diabetes include polycystic ovarian syndrome and steroid induced insulin resistance.

The complications of conditions associated with diabetes mellitus encompassed herein includes renal disease, especially renal disease associated with the development of Type II diabetes including diabetic nephropathy, glomerulonephritis, glomerular sclerosis, nephrotic syndrome, hypertensive nephrosclerosis and end stage renal disease.

As mentioned above the compound of the invention has useful therapeutic properties: The present invention accordingly provides Hydrochloride Hydrate for use as an active therapeutic substance.

More particularly, the present invention provides Hydrochloride Hydrate for use in the treatment and/or prophylaxis of diabetes mellitus, conditions associated with diabetes mellitus and certain complications thereof.

Hydrochloride Hydrate may be administered per se or, preferably, as a pharmaceutical composition also comprising a pharmaceutically acceptable carrier. The formulation of Hydrochloride Hydrate is generally as disclosed for Compound (I) in the above mentioned publications.

Accordingly, the present invention also provides a pharmaceutical composition comprising Hydrochloride Hydrate and a pharmaceutically acceptable carrier therefor.

Hydrochloride Hydrate is normally administered in unit dosage form.

The active compound may be administered by any suitable route but usually by the oral or parenteral routes. For such use, the compound will normally be employed in the form of a pharmaceutical composition in association with a pharmaceutical carrier, diluent and/or excipient, although the exact form of the composition will naturally depend on the mode of administration.

Compositions are prepared by admixture and are suitably adapted for oral, parenteral or topical administration, and as such may be in the form of tablets, capsules, oral liquid preparations, powders, granules, lozenges, pastilles, reconstitutable powders, injectable and infusable solutions or suspensions, suppositories and transdermal devices. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

Solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the active compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the active compound is suspended in the vehicle instead of being dissolved and sterilised by exposure to ethylene oxide before suspending in the sterile vehicle. Advantageously, a surfactant or wetting agent is included in the composition to facilitate uniform distribution of the active compound.

In addition, the Hydrochloride Hydrate may be used in combination with other antidiabetic agents such as insulin secretagogues, for example sulphonylureas, biguanides, such as metformin, alpha glucosidase inhibitors, such as acarbose, beta agonists, and insulin such as those disclosed in WO98/57649, WO98/57634 , WO98/57635 or WO98/57636. The other antidiabetic agents, the amounts thereof and methods of administration are as described in the above mentioned publications. The formulation of the Hydrochloride Hydrate and dosages thereof in said combinations are generally as disclosed for Compound (I) in the above mentioned publications.

As is common practice, the compositions will usually be accompanied by written or printed directions for use in the medical treatment concerned.

As used herein the term 'pharmaceutically acceptable' embraces compounds, compositions and ingredients for both human and veterinary use: for example the term 'pharmaceutically acceptable salt' embraces a veterinarily acceptable salt.

The present invention further provides a method for the treatment and/or prophylaxis of diabetes mellitus, conditions associated with diabetes mellitus and certain complications thereof, in a human or non-human mammal which comprises administering an effective, non-toxic, amount of Hydrochloride Hydrate to a human or non-human mammal in need thereof.

Conveniently, the active ingredient may be administered as a pharmaceutical composition hereinbefore defined, and this forms a particular aspect of the present invention.

In a further aspect the present invention provides the use of Hydrochloride Hydrate for the manufacture of a medicament for the treatment and/or prophylaxis of diabetes mellitus, conditions associated with diabetes mellitus and certain complications thereof.

In the treatment and/or prophylaxis of diabetes mellitus, conditions associated with diabetes mellitus and certain complications thereof Hydrochloride Hydrate may be taken in doses, such as those disclosed in the above mentioned publications

No adverse toxicological effects are indicated in the above mentioned treatments for the compounds of the invention.

The following example illustrates the invention but does not limit it in any way.

Example 1: Preparation of 5-[4-[2-(N-methyl-N-(2-pyridyl)amino)ethoxy]benzyl]thiazolidine-2,4-dione, hydrochloride monohydrate (Hydrochloride Hydrate): To a solution of 5-[4-[2-(N-methyl-N-(2-pyridyl)amino)ethoxy]benzyl]thiazolidine-2,4-dione (10g) in acetic acid (100 g) was added concentrated hydrochloric acid (10 ml). After 15 mins the clear solution was treated with diethyl ether (150 ml). The supernatant liquid was decanted, and the residue stirred with fresh diethyl ether (100 ml). The liquid was again decanted, and the thick oil stirred with fresh diethyl ether (50 ml). The product was filtered and washed with diethyl ether and dried under vacuum at 50C to give the title compound-(7.67 g, 66.5%), melting point - 100-3°C with resolidification and remelting at 150-4°C.

Example 2: Concentrated hydrochloric acid (1.0 ml) was added to a stirred suspension of 5-[4-[2-(N-methyl-N-(2-pyridyl)amino)ethoxy]benzyl]thiazolidine-2,4-dione (4.0 g) in a mixture of propan-2-ol (100 ml) and water (10 ml) at 21°C. The mixture was warmed to 25°C and stirred at this temperature for a period of 1 hour. The product was collected by filtration, washed with propan-2-ol (20 ml) and dried under vacuum to give Hydrochloride Hydrate_(3.8 g).

Example 3: A mixture of 5-[4-[2-(N-methyl-N-(2-pyridyl)amino)ethoxy]benzyl]thiazolidine-2,4-dione (4.0 g), propan-2-ol (100 ml) and water (10 ml) was warmed to 75°C with stirring. Aqueous hydrochloric acid (2.3M, 5.0 ml) was added to the stirred suspension and the resulting solution was cooled to 21°C over a period of 1 hour. The mixture was seeded with crystals of Hydrochloride Hydrate and stirred for 30 minutes. The product was collected by filtration to give Hydrochloride Hydrate as a white crystalline solid (3.9 g).

Example 4 : Concentrated hydrochloric acid (2.0ml) was added to a mixture of 5-[4-[2-(N-methyl-N-(2-pyridyl)amino)ethoxy]benzyl]thiazolidine-2,4-dione5-[4-[2-(N-methyl-N-(2-pyridyl)amino)ethoxy]benzyl]thiazolidine-2,4-dione (2.0 g) and acetonitrile (20 ml) and the mixture stirred at 21°C until a clear solution was observed. Diethyl ether (15 ml) was added to the stirred solution followed by seed crystals of Hydrochloride Hydrate_(20 mg). After stirring for 5 minutes, diethyl ether (15 ml) was added and stirring continued for 1 hour. The product was collected by filtration, washed with diethyl ether/acetonitrile (2:1, 20 ml) and dried under vacuum for 5 hours to give Hydrochloride Hydrate as a white crystalline solid (1.9 g).

Example 5: A mixture of 5-[4-[2-(N-methyl-N-(2-pyridyl)amino)ethoxy]benzyl]thiazolidine-2,4-dione (10.0 g) and propan-2-ol (140 ml) was stirred and warmed to 60°C. Aqueous hydrochloric acid (4M, 14 ml) was added to the stirred mixture and the temperature raised to 70°C and maintained at this temperature for 10 minutes. The resulting clear solution was cooled to 21°C over a period of approximately 1 hour. The product was collected by filtration, washed with propan-2-ol (30 ml) and dried under vacuum over phosphorus pentoxide for 20 hours to give Hydrochloride Hydrate as a white crystalline solid (10.4 g).

**CHARACTERISING DATA:** The following characterising data were generated for Hydrochloride Hydrate:

### A Water content

This was determined as 4.5% w/w using a Karl Fischer apparatus (theory for monohydrate 4.37% w/w).

### B Ionic Chlorine

This was determined as 8.7% w/w (theory for monohydrate 8.61% w/w).

### C Infrared

The infrared absorption spectrum of a mineral oil dispersion of Hydrochloride Hydrate was obtained using a Nicolet 710 FT-IR spectrometer at 2 cm⁻¹ resolution. Data were digitised at 1 cm⁻¹ intervals. The spectrum obtained is shown in Figure I. Peak positions are as follows: 3358, 3124, 2764, 1762, 1747, 1707, 1644, 1614, 1587, 1544, 1511, 1414, 1333, 1302, 1268, 1245, 1181, 1154, 1142, 1108, 1075, 1054, 1033, 1012, 988, 938, 905, 859, 833, 817, 760, 738, 716, 663, 652, 636, 620, 605, 564, 540, 525 and 505cm⁻¹.

### B X-Ray Powder Diffraction (XRPD)

The XRPD pattern of Hydrochloride Hydrate is shown below in Figure II and a summary of the XRPD angles and calculated lattice spacings characteristic of Hydrochloride Hydrate is given in Table I.
A Philips PW1710 X-ray powder diffractometer (Cu X-ray source) was used to generate the pattern using the following acquisition conditions:

| | |
|---|---|
| Tube anode | Cu |
| Generator tension | 40 kV |
| Generator current | 30 mA |
| Start angle | 3.5 °2θ |
| End angle | 35.0 °2θ |
| Step size | 0.020 °2θ |
| Time per step | 2.3 s |

**Table I.**

| X-Ray Powder Diffraction Angles and Calculated Lattice Spacings Characteristic of Hydrochloride Hydrate. | |
|---|---|
| Diffraction Angle (°2θ) | Lattice Spacing (Angstroms) |
| 8.8 | 10.06 |
| 12.1 | 7.31 |
| 14.1 | 6.30 |
| 14.3 | 6.17 |
| 15.0 | 5.90 |
| 16.5 | 5.38 |
| 17.7 | 5.01 |
| 19.1 | 4.62 |
| 19.9 | 4.46 |
| 20.7 | 4.28 |
| 21.3 | 4.17 |
| 21.7 | 4.09 |
| 23.0 | 3.86 |
| 23.8 | 3.73 |
| 24.3 | 3.66 |
| 24.9 | 3.57 |
| 25.6 | 3.48 |
| 27.0 | 3.30 |
| 27.6 | 3.23 |
| 28.9 | 3.08 |
| 30.0 | 2.97 |
| 31.4 | 2.85 |
| 32.2 | 2.78 |
| 33.1 | 2.71 |

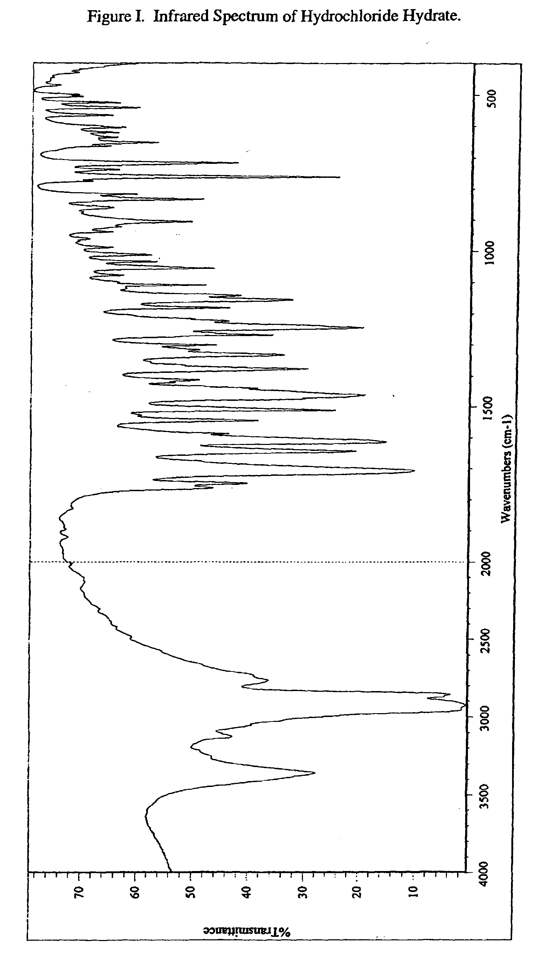

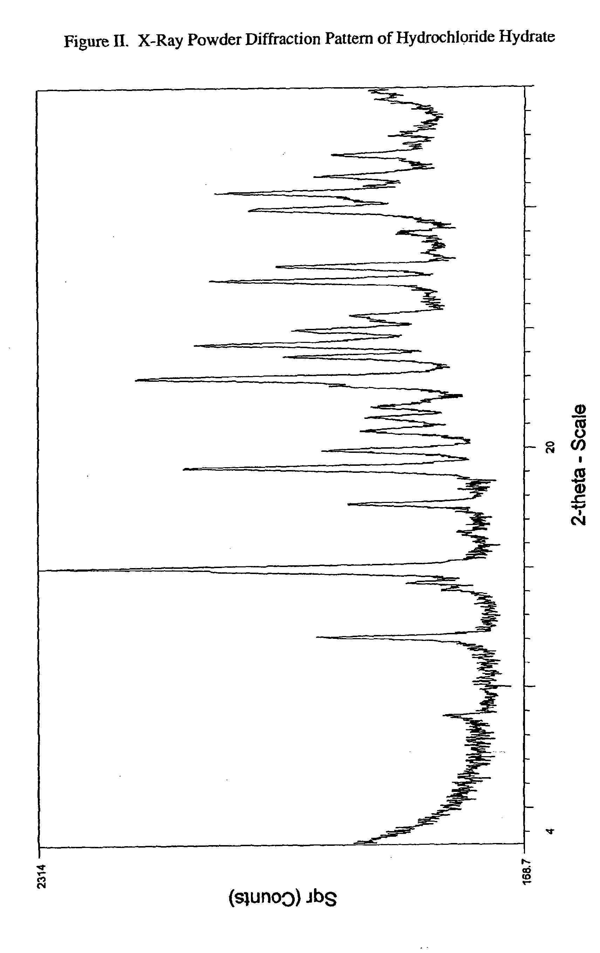

## Claims

1. 5-[4-[2-(N-methyl-N-(2-pyridyl)amino)ethoxy]benzyl]thiazolidine-2,4-dione, hydrochloride monohydrate **characterised in that** it:
(i) provides an infra red spectrum containing peaks at 3358, 2764, 1245, 833 and 760cm⁻¹; and/or
(ii) provides an X-ray powder diffraction (XRPD) pattern containing peaks at 15.0, 17.7, 23.0, 30.0 and 31.4 °2θ.

2. A hydrate according to claim 1, which provides an infra red spectrum substantially in accordance with Figure I.

3. A hydrate according to claim 1 or claim 2, which provides an X-ray powder diffraction (XRPD) pattern substantially in accordance with Figure II

4. A hydrate according to any one of claims 1 to 3, in isolated form.

5. A hydrate according to any one of claims 1 to 4, in pure form.

6. A hydrate according to any one of claims 1 to 5, in crystalline form.

7. A process for preparing a hydrate according to claim 1, **characterised in that** either:
(a) 5-[4-[2-(N-methyl-N-(2-pyridyl)amino)ethoxy]benzyl]thiazolidine-2,4-dione (Compound I) is treated with a source of hydrochloride ions in acetic acid as solvent; or
(b) a suspension of Compound (I) in an aqueous organic solvent is treated with a source of hydrochloride ions at a temperature in the range of from 20°C to 30°C; or
(c) a solution of Compound (I) in an organic solvent is reacted with a source of hydrochloride ions to provide substantially dissolved reactants, crystallisation is then induced to provide the Hydrochloride Hydrate; and thereafter the required compound is recovered.

8. A pharmaceutical composition comprising an effective, non-toxic amount of a hydrate according to claim 1 and a pharmaceutically acceptable carrier therefor.

9. A hydrate according to claim 1, for use as an active therapeutic substance.

10. A hydrate according to claim 1, for use in the treatment and/or prophylaxis of diabetes mellitus, conditions associated with diabetes mellitus and certain complications thereof.

11. The use of hydrate for the manufacture of a medicament for the treatment and/or prophylaxis of diabetes mellitus, conditions associated with diabetes mellitus and certain complications thereof.

12. A method for the treatment and/or prophylaxis of diabetes mellitus, conditions associated with diabetes mellitus and certain complications thereof, in a human or non-human mammal which comprises administering an effective, non-toxic, amount of hydrate to a human or non-human mammal in need thereof.
